# EUROPEAN PATENT APPLICATION

(11) **EP 1 891 983 A1**
(43) Date of publication of application: **27.02.2008**
(21) Application number: 04808698.7
(22) Date of filing: 15.12.2004
(51) Int. Cl.: A61L 15/34, A61L 15/62, A61L 15/28, A61F 13/15

(54) **IMPROVEMENTS TO ECOLOGICAL AND BIODEGRADABLE ABSORBENT ARTICLES**

(71) Applicant: Flores Gonzales, Estela Concepcion, Santiago de Querétaro, Querétaro 76020 (MX)
(72) Inventor: Flores Gonzales, Estela Concepcion, Santiago de Querétaro, Querétaro 76020 (MX)
(74) Representative: Gonzalez Gomez, Maria Virtudes
(86) International application number: PCT/MX2004/000092
(87) International publication number: WO 2006/065110

(57) **Abstract**

The invention is a new natural impermeable waterproof instrument, a new femenine absorber cushion and a new independent impermeable cover. All for the use of the hygienic ecological and biodegradable absorber towels or pads. PCT/MX02/00041, PCT/MX02/00042 y PCT/MX03/00099.

The new natural impermeable waterproof instrument, is a semisolid, partially polymerized, and with high-viscosity fat macromolecules suspension. When a thin layer of the suspension is spread on the fabric-like-paper, it satisfactory waterproofs the paper. The layer also allows the paper to conserve its own flexibility. This is why the layer is ideal for the ecological absorbers (pads) because it does not permit that the body fluids pass trough it.

The feminine absorber (pad) cushion holds and absorbs the menstrual fluids that move towards the back of the body when a woman lays on her back Also, a new impermeable (waterproof) cover-layer is placed under any of the ecological and biodegradable feminine absorbers (pads) to have extra and total protection. The new independent impermeable cover is made of a plastic layer that is reusable, washable and recyclable.

## Description

The invention is a new natural impermeable waterproof instrument, a new femenine absorber cushion and a new independent impermeable cover. All for the use of the hygienic ecological and biodegradable absorber towels or pads. PCT/MX02/00041, PCT/MX02/00042 y PCT/MX03/00099.

The new natural impermeable waterproof instrument, is a semisolid, partially polymerized, and with high-viscosity fat macromolecules suspension. When a thin layer of the suspension is spread on the fabric-like-paper, it satisfactory waterproofs the paper. The layer also allows the paper to conserve its own flexibility. This is why the layer is ideal for the ecological absorbers (pads) because it does not permit that the body fluids pass trough it.

The feminine absorber (pad) cushion holds and absorbs the menstrual fluids that move towards the back of the body when a woman lays on her back

Also, a new impermeable (waterproof) cover-layer is placed under any of the ecological and biodegradable feminine absorbers (pads) to have extra and total protection. The new independent impermeable cover is made of a plastic layer that is reusable, washable and recyclable.

The new natural (impermeable) waterproof suspension is a mix of vegetable fat that can be made of one or many of the natural vegetable oils. The suspension must be submitted to a hydrogenation process. The vegetable oils can be made of com, saffron (carthamus), sunflowers, sesame seeds, coconut, pumpkin, and peanuts.

In the suspension one must add a vegetable absorber polymer or rubber, such as guar, carragenina, tragacanta, karaya, vetch, arabiga (arabicus), acacia beans (locust tree), Irish moss etc.

Also any emulsifier used in the food industry can be used as a substitute of the vegetable absorber polymer: like gelatin (jelly), sodium algin, pectin, methylcellulose pectin or starch (com, potato or wheat modified starch).
The function of this new element is to link the fat macromolecules and to give them cohesion mostly at the moment of receiving humidity.

In the suspension one must add too, a small portion of antioxidant or preservative such as (alfa tocoferol, sodium benzoate, sodium carbonate, sodium chloride) or any other element that can delay the oxidation and the nitrogenous ionization processes. An antioxidant or preservative must be added for the use of the product on extremely hot climates.

Any process of hydrogenation acts in a different way depending on the type of oil and on its physical and chemical characteristics. The oil can form polymeric macromolecules increasing its viscosity; in some cases it can solidify itself, or even modify its density reinforcing its impermeability as a consequence.

The new feminine absorber (pad) cushion is an option to avoid the use of nighttime menstrual pads which are too long and have too much volume making them uncomfortable or noticeable by others when used during the day with stretch or transparent clothing

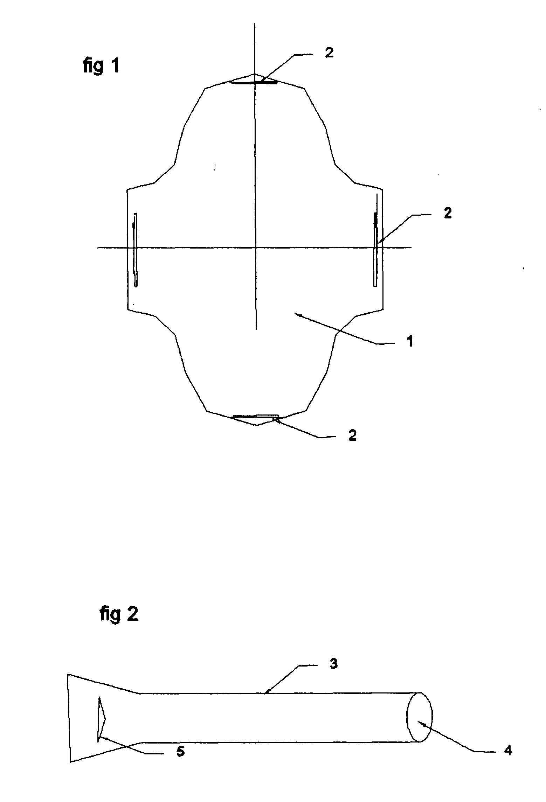

The new nighttime feminine absorber cushion is a small; long tubular, and narrow sanitary towel (pad) made of rolled and re-rolled fabric-like-paper, or filled with cellulose pulp (FIG 2).

It has one flat or semi-flat end, with a cut (simple loop hole) through which the back fastener of the ecological underwear passes. The absorber is placed to stay between the gluteus, and in this way, absorb the menstrual fluids that move towards the back of the body when a woman lays on her back letting her sleep or rest safely and calmly.

We now count on the protection of the ecological underwear, the feminine hygienic, ecological and biodegradable absorber, the new independent impermeable cover and the new feminine absorber cushion.

In the case of the ecological and biodegradable diaper we count on the total protection of the fabric and the plastic diaper. Now also, the feminine ecological and biodegradable absorbers (pads) have an additional, simple and safe, protection. This protection guarantees the secure use of all the absorbers, since they are made of completely natural, fine and delicate materials.

**The new independent and impermeable cover** is made of a plastic layer that can be made of Poly Vinyl Chloride (PVC). It has the same shape and size of the absorber (pad) wished to protect (FIG.1.) and has cuts (or simple loop holes) that coincide with the loop holes of the absorber and the elastic fasteners of the ecological underwear. Both are fixed together by passing the elastic fasteners through the loop holes and snapping the clasps of the fasteners. The clasps can be opened at any time to remove the absorber or to check it. The cover always stays in good condition because it is reusable, washable and recyclable.

### PARTS

1.- Plastic cover that can be made of Poly vinyl Choride (PVC)
2.- Cuts or loop holes.
3.- Soft paper cover.
4.- Natural and absorbent material, that can be cellulose pulp or soft paper.
5.- One cut or loop hole

Having described in explicit form my invention, I consider it to be an innovation (newness) and therefore claim as my esclusive property the contents or the following clauses.

## Claims

1. The new natural impermeable waterproof instrument is a semisolid, partially polymerized, and with high viscosity fat macromolecules suspension, used in various ways- especially for those of ecological significance.

2. The impermeable natural cover, for all the hygienic, ecologic, and biodegradable absorbers, is made of fabric-like-paper covered with a thin and uniform layer of the natural waterproof suspension.

3. The new natural waterproof suspension is a mix of fat (that can be animal as well as butter or pork fat or vegetable which is more ideal), plus some vegetable absorber polymer or rubber, plus a small portion of some antioxidant or preservative.

4. The vegetable fat can be made of one or more of the vegetable type oils like com, saffron (carthamus), sunflowers, sesame seeds, coconut, pumpkin, and peanuts. The suspension must be submitted to a hydrogenation process.

5. The vegetable absorber polymer or rubber in the mix of the new natural waterproof suspension can be made of guar, carragenina, tragacanta, karaya, vetch, arabiga (arabicus), acacia beans (locust tree), Irish moss etc

6. Also any emulsifier used in the food industry can be used as a substitute of the vegetable absorber polymer in the mix of the new natural waterproof suspension: like gelatin (jelly), sodium algin, pectin, methyl cellulose pectin, or starch (com, potato or wheat modified starch).

7. The use of an antioxidant or preservative in the mix of the new natural waterproof suspension can be such as (alfa tocoferol, sodium benzoate, sodium carbonate, sodium chloride) or any other element that can delay the oxidation and the nitrogenous ionization processes. An antioxidant or preservative must be added for the use of the product on extremely hot climates.

8. The new independent impermeable cover made of a plastic layer placed under the feminine hygienic, ecological and biodegradable absorber is fixed with the elastic fasteners of the ecological underwear.

9. The feminine absorber cushion made of a rolled and re-rolled fabric-like-paper or filled with cellulose pulp is fixed with the back fastener of the ecological underwear to absorb the menstrual fluids that move towards the back when a woman lies on her back.

10. The system of elastic fasteners with clasps or buttons placed strategically in the plastic or fabric diaper or in the ecological underwear is to fix all the hygienic, ecological and biodegradable absorbers using the cuts or loop holes.
